# EUROPÄISCHE PATENTSCHRIFT

(11) **EP 2 729 078 B1**
(45) Veröffentlichungstag und Bekanntmachung des Hinweises auf die Patenterteilung: **28.12.2016**
(21) Anmeldenummer: 12730535.7
(22) Anmeldetag: 28.06.2012
(51) Int. Cl.: A61B 17/435, A61D 19/04, A61B 10/02, A61B 17/00, A61B 17/34

(54) **PUNKTIONSVORRICHTUNG FÜR DIE ENTNAHME ORGANISCHER PROBEN**
PUNCTURING DEVICE FOR REMOVING ORGANIC SAMPLES
DISPOSITIF DE PONCTION POUR LE PRÉLÈVEMENT D'ÉCHANTILLONS ORGANIQUES

(30) Priorität: 04.07.2011 AT 9782011
(43) Veröffentlichungstag der Anmeldung: 14.05.2014
(73) Patentinhaber: Steiner, Hans-Peter, 8010 Graz (AT)
(72) Erfinder: Steiner, Hans-Peter, 8010 Graz (AT)
(74) Vertreter: Babeluk, Michael
(86) Internationale Anmeldenummer: PCT/EP2012/062536
(87) Internationale Veröffentlichungsnummer: WO 2013/004578

(56) Entgegenhaltungen:
- EP-A2- 1 967 147
- WO-A1-2009/150399
- US-A- 5 160 319

## Beschreibung

Die Erfindung betrifft eine Punktionsvorrichtung für die Entnahme organischer Proben, beispielsweise einer Eizelle für die In-Vitro-Fertilisierung, mit einer zu einer Nadelspitze zulaufenden Punktionskanüle, wobei das Lumen der Punktionskanüle als Saugkanal ausgebildet ist, sowie mit einer Spülleitung, welche zur Zufuhr eines Spülmittels dient, wobei im Anschluss an die Nadelspitze der Punktionskanüle ein einlumiger Punktionsbereich ausgebildet ist, an welchen ausgehend von einer Verbindungsstelle, in welcher die Spülleitung flüssigkeitsdicht an der Punktionskanüle befestigt ist, ein doppellumiger Bereich anschließt, wobei am distalen Ende der Spülleitung zumindest eine Übertrittsöffnung in den Saugkanal der Punktionskanüle vorgesehen ist.

Im Rahmen der Sterilitätsbehandlung mit Hilfe der klassischen In-Vitro-Fertilisierung (IVF) werden die Eizellen durch Punktion des Eibläschens (Follikel) unmittelbar vor dem zu erwartenden Eisprung gewonnen. Mittels Hormonen wird die Reifung der Eizellen unterstützt, wodurch gleichzeitig mehrere Eizellen heranreifen und die Erfolgschancen der IVF erhöht werden. Meist werden bei der Follikel-Punktion Punktionsnadeln mit einem Außendurchmesser von 1,4 mm bis 1,5 mm und einer Länge von 30 cm verwendet, wobei pro Eibläschen (Durchmesser bis zu 2,5 cm) etwa 5 ml Follikelflüssigkeit abgesaugt werden.

Zum Teil können allerdings durch die Hormonbehandlung Schwierigkeiten auftreten, so dass für Patientinnen mit dem sogenannten PCO-Syndrom (Polyzystische Ovarien) die lebensbedrohliche Komplikation eines sogenannten ovariellen Hyperstimulationssyndroms(OHSS) auftreten kann. Ein neues Verfahren, die sogenannte In-Vitro-Maturation (IVM),verhindert diese Komplikation.

Bei der IVM wird ohne vorherige Hormongabe in einem spontanen Zyklus bereits bei einer Follikelgröße von 8 mm bis 10 mm meist zwischen dem 8. und 10. Zyklustag die Follikelreifung hormonell ausgelöst, wobei nach ca. 36 bis 38 Stunden alle vorhandenen Eibläschen ab einer Größe von 3 mm bis 4 mm punktiert und unter Ultraschallkontrolle abgesaugt werden. Die Eizellen werden dann im Brutschrank 24 Stunden nachgereift und danach im Falle des Vorhandenseins eines Polkörperchens mittels der ICSI-Methode fertilisiert.

Aufgrund der geringen Follikelgröße bei der IVM sind allerdings viele der bekannten Punktionssets zur Gewinnung der Eizelle ungeeignet.

Es ist bekannt Punktionsvorrichtungen mit einer Spülvorrichtung zu verwenden, wobei beispielsweise mit Hilfe eines Dreiwege-Ventils, welches an eine einlumige Punktionsnadel anschließt, je nach Stellung des Ventils gespült oder aspiriert werden kann. Ein Nachteil derartiger Systeme besteht darin, dass generell ein großes Totvolumen vorhanden ist, welches dem Volumen der Punktionsvorrichtung von der Kanülenspitze bis zum Dreiwege-Ventil entspricht.

Zur Minimierung des Totvolumens wurden daher auch doppellumige Punktionskanülen eingesetzt, bei welchen eine Kanüle zur Aspiration und eine zum Spülen des Follikels dient. Der Nachteil bei doppellumigen Punktionssets besteht darin, dass der Gesamtdurchmesser relativ groß ist und daher derartige Sets für die IVM kaum geeignet sind.

Eine doppellumige Punktionskanüle zur Follikelpunktion ist beispielsweise aus der DE 35 22 782 A1 bekannt geworden, welche aus einem Außenrohr mit im Wesentlichen kreisförmigen Querschnitt und einem Innenrohr besteht, dessen Außenwand in einem großen Umfangsbereich an der Innenwand des Außenrohrs anliegt. Das Innenrohr weist in einem kleinen Bereich mit einem Umfangswinkel kleiner 90° eine Einwölbung auf, welche in diesem Bereich zum Außenrohr einen Abstand bildet und einen Spülkanal bildet. Der verbleibende Saugkanal im Innenrohr weist dadurch allerdings eine den freien Durchtritt der durch Punktion gewonnenen Eizellen behindernde Einwölbung auf. Weiters besteht durch diese Einwölbung an der Eintrittsöffnung im Bereich der Nadelspitze eine Kante, welche ebenfalls störend für die Aufnahme organischer Proben ist.

Schließlich ist aus der EP 1 967 147 B1 eine Punktionsvorrichtung für die Entnahme einer organischen Probe bekannt, bei welcher im Anschluss an die Nadelspitze ein dünnerer einlumiger Punktionsbereich ausgebildet ist, an welchen ausgehend von einer Verbindungsstelle, in welcher eine Spülleitung flüssigkeitsdicht an der Punktionskanüle befestigt ist, ein doppellumiger Bereich anschließt, in welchem die Punktionskanüle in der Spülleitung verläuft, so dass zwischen der Außenwand der Punktionskanüle und der Innenwand der Spülleitung ein Spülkanal ausgebildet ist, welcher an dessen distalem Ende zumindest eine Übertrittsöffnung in den Saugkanal der Punktionskanüle aufweist. Der einlumige Punktionsbereich der Kanüle kann zwar im Vergleich mit den bekannten, doppellumigen Nadeln mit einem kleineren Außendurchmesser hergestellt werden, wodurch die Follikelpunktion aufgrund der geringeren Nadeldicke wesentlich weniger schmerzhaft ist, nachteiligerweise ist jedoch bei einem kleinen Nadeldurchmesser die Übertrittsöffnung zum Spülkanal mit einem für optimale Spülergebnisse ausreichenden Durchmesser nur sehr aufwendig herzustellen. Durch eine Übertrittsöffnung mit einem im Verhältnis zum Nadeldurchmesser großen Innendurchmesser kann es zudem zu einer Schwächung der Nadelstruktur in diesem Bereich kommen, was zu einer Verbiegung oder einem Bruch der Punktionskanüle führen kann.

Die EP 1 967 147 B1 offenbart die Merkmale des Oberbegriffs von Anspruch 1.

Aufgabe der Erfindung ist es, eine Punktionsvorrichtung für die Entnahme einer organischen Probe, beispielsweise einer Eizelle, derart weiter zu entwickeln, dass deren Anwendung neben der herkömmlichen IVF auch vorteilhaft für die In-Vitro-Maturation (IVM) eingesetzt werden kann, wobei ein möglichst einfach und kostengünstig herzustellendes Punktions-Set realisiert werden soll.

Diese Aufgabe wird erfindungsgemäß dadurch gelöst, dass die Punktionskanüle im Punktionsbereich einen kleineren Durchmesser aufweist als im proximal anschließenden doppellumigen Bereich, wobei die zumindest eine Übertrittsöffnung am distalen Ende des doppellumigen Bereichs angeordnet ist. Der Bereich mit der Übertrittsöffnung weist somit einen größeren Durchmesser auf, als der einlumige Bereich mit der Nadelspitze, sodass für die Punktion Nadeln mit kleinsten Außendurchmessern verwendet werden können.

Bevorzugt besteht dabei die Punktionskanüle im Wesentlichen aus einer dünnen Punktionsnadel (beispielsweise eine 22 Gauge Nadel) und einer proximal anschließenden Anschlusskanüle (beispielsweise eine 18 Gauge Kanüle), wobei das proximale Ende der Punktionsnadel in die Anschlusskanüle ragt. Dabei ist der Außendurchmesser der Punktionsnadel (z.B. 0.72 mm) an den Innendurchmesser der Anschlusskanüle (0.84 mm) angepasst.

Das proximale Ende der Punktionsnadel kann erfindungsgemäß durch Klemmen, Kleben oder Schweißen (z.B. Laserschweißen) in der Anschlusskanüle befestigt werden.

Bei einer Klebeverbindung kann die Punktionsnadel aus Stahl und die Anschlusskanüle aus Kunststoff hergestellt sein, was die Produktionskosten wesentlich verringert.

Weiters ist es möglich die Anschlusskanüle auf das Ende der Punktionsnadel aufzuschrumpfen. Dabei weist die Punktionsnadel einen Außendurchmesser auf der geringfügig größer ist als der Innendurchmesser der Anschlusskanüle. Nach einer entsprechenden Wärmebehandlung der Anschlusskanüle und/oder Kältebehandlung (beispielsweise mit flüssigem Stickstoff) der Punktionsnadel werden die beiden Teile zusammengefügt und sind nach dem Temperaturausgleich fest miteinander verbunden.

Die Spülleitung der Punktionsvorrichtung kann als flexibler Spülschlauch ausgebildet sein, welcher an der Verbindungsstelle zur Punktionskanüle eine Durchmesserverengung, eine Schrumpfschlauchverbindung oder eine Klebeverbindung aufweist.

Die Erfindung wird im Folgenden anhand von Ausführungsbeispielen näher erläutert. Es zeigen:
- Fig. 1: eine erfindungsgemäße Punktionsvorrichtung in einem Längsschnitt; sowie
- Fig. 2: ein Detail II aus Fig. 1 in einer vergrößerten Darstellung.

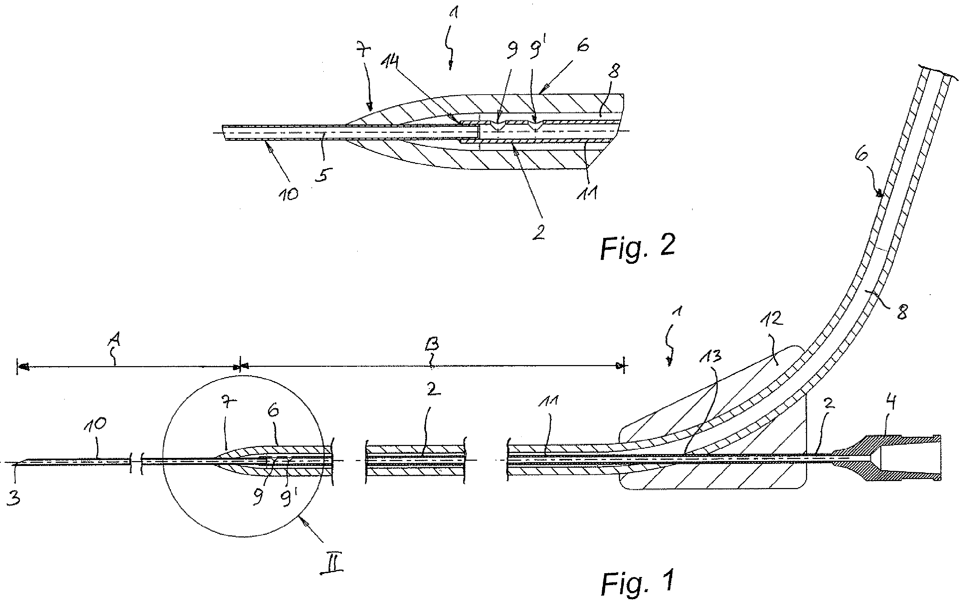

Die Punktionsvorrichtung 1 gemäß Fig. 1 samt Detail gemäß Fig. 2 dient für die Entnahme einer organischen Probe und weist eine Punktionskanüle 2 mit einer geschliffenen Nadelspitze 3 am distalen Ende auf, wobei an deren proximalen Ende ein Luer-Anschluss 4 angeordnet ist, mit welchem über einen Verbindungsschlauch ein Aufnahmebehälter für die organische Probe, beispielsweise eine Eizelle, angeschlossen werden kann. Der Aufnahmebehälter ist - wie aus der eingangs zitierten EP 1 967 147 B1 bekannt - mit einer Vakuumquelle verbunden, um im Lumen der als Saugkanal 5 ausgeführten Punktionskanüle 2 den für die Probengewinnung nötigen Unterdruck erzeugen zu können.

Weiters ist eine als flexibler Spülschlauch ausgeführte Spülleitung 6 vorgesehen, mit welcher ein Spülmittel beispielsweise zur Follikelspülung eingebracht werden kann. Als Spülmittelbehälter kann beispielsweise eine Kolbenspritze mit der Spülleitung 6 verbunden sein, wobei durch eine gleichzeitige, aufeinander abgestimmte Saug- und Spültätigkeit ein optimales Ergebnis bei der Follikelspülung erzielt werden (siehe EP 1 967 147 B1).

Die Spülleitung 6 umfasst die Punktionskanüle 2 und ist an einer von der Nadelspitze 3 ca. 6 cm bis 9 cm beabstandeten Verbindungsstelle 7 flüssigkeitsdicht an dieser befestigt. Es entsteht so zwischen der Außenwand der Punktionskanüle 2 und der Innenwand der Spülleitung 6 ein kreisringförmiger Abschnitt des Spülkanals 8, welcher im Bereich des distalen Endes zwei Übertrittsöffnungen 9, 9' in den Saugkanal 5 aufweist (siehe Fig. 2). Die Punktionskanüle 2 weist im Punktionsbereich A einen kleineren Durchmesser auf als im proximal anschließenden doppellumigen Bereich B, wobei die Übertrittsöffnungen 9, 9' am distalen Ende des doppellumigen Bereichs B angeordnet sind.

Durch die erfindungsgemäße Ausführung besteht im Anschluss an die Nadelspitze 3 ein dünner, einlumiger Punktionsbereich A, welcher sich hervorragend für die Eizellengewinnung eignet, wobei die Übertrittsöffnungen 9, 9' in einem Bereich mit größerem Durchmesser angeordnet sind und dadurch entsprechend größere Querschnitte realisierbar sind.

Wie in Fig. 1 und Fig. 2 dargestellt, besteht die Punktionskanüle 2 im Wesentlichen aus einer relativ dünnen Punktionsnadel 10 mit der Nadelspitze 3 und einer proximal anschließenden Anschlusskanüle 11, wobei das proximale Ende der Punktionsnadel 10 in die Anschlusskanüle 11 ragt. Der Außendurchmesser der Punktionsnadel 10 kann so optimal an den Innendurchmesser der Anschlusskanüle 11 angepasst und durch Klemmen, Schrumpfen, Kleben oder Schweißen befestigt werden.

Beispielsweise kann die Punktionsnadel 10 aus Metall, vorzugsweise aus einer Stahllegierung, und die Anschlusskanüle 11 aus Kunststoff bestehen. Es ist auch möglich, eine Punktionsnadel 10 und eine Anschlusskanüle 11 aus Metall, vorzugsweise aus einer Stahllegierung, zu verwenden und diese durch Schweißen, beispielsweise durch Laserschweißen 14, zu verbinden.

Die Anschlusskanüle 11 kann im distalen Bereich zwei oder mehrere Übertrittsöffnungen 9, 9' aufweisen, die bevorzugt in axialer Richtung hintereinander angeordnet sind. Dadurch wird der Strömungsquerschnitt der Übertrittsöffnungen erhöht, ohne die Nadelstruktur in diesem Bereich allzu sehr zu schwächen.

Die Spülleitung 6 ist bevorzugt als flexibler Spülschlauch ausgebildet, welcher an der Verbindungsstelle 7 zur Punktionskanüle 2 eine Durchmesserverengung, eine Schrumpfschlauchverbindung oder eine Klebeverbindung aufweist. Die Punktionskanüle 2 kann ein Griffelement 12 aufweisen und in diesem Bereich durch eine Einstichöffnung 13 in das Innere der Spülleitung 6 eintreten. Die Punktionskanüle 2 wird durch den flexiblen Spülschlauch an der Einstichöffnung 13 flüssigkeitsdicht abgedichtet.

## Patentansprüche

1. Punktionsvorrichtung (1) für die Entnahme organischer Proben, beispielsweise einer Eizelle für die In-Vitro-Fertilisierung, mit einer zu einer Nadelspitze (3) zulaufenden Punktionskanüle (2), wobei das Lumen der Punktionskanüle (2) als Saugkanal (5) ausgebildet ist, sowie mit einer Spülleitung (6), welche zur Zufuhr eines Spülmittels dient, wobei im Anschluss an die Nadelspitze (3) der Punktionskanüle (2) ein einlumiger Punktionsbereich (A) ausgebildet ist, in dem die Punktionskanüle ein einziges Lumen aufweist und an welchen ausgehend von einer Verbindungsstelle (7), in welcher die Spülleitung (6) flüssigkeitsdicht an der Punktionskanüle (2) befestigt ist, ein doppellumiger Bereich (B) anschließt, wobei am distalen Ende der Spülleitung (6) zumindest eine Übertrittsöffnung (9) in den Saugkanal (5) der Punktionskanüle (2) vorgesehen ist, **dadurch gekennzeichnet, dass** die Punktionskanüle (2) im Punktionsbereich (A) einen kleineren Durchmesser aufweist als im proximal anschließenden doppellumigen Bereich (B), wobei die zumindest eine Übertrittsöffnung (9) am distalen Ende des doppellumigen Bereichs (B) angeordnet ist.

2. Punktionsvorrichtung (1) nach Anspruch 1, **dadurch gekennzeichnet, dass** die Punktionskanüle (2) im Wesentlichen aus einer dünnen Punktionsnadel (10) mit der Nadelspitze (3) und einer proximal anschließenden Anschlusskanüle (11) besteht, wobei das proximale Ende der Punktionsnadel (10) in die Anschlusskanüle (11) ragt.

3. Punktionsvorrichtung (1) nach Anspruch 2, **dadurch gekennzeichnet, dass** der Außendurchmesser der Punktionsnadel (10) an den Innendurchmesser der Anschlusskanüle (11) angepasst ist.

4. Punktionsvorrichtung (1) nach Anspruch 2 oder 3, **dadurch gekennzeichnet, dass** das proximale Ende der Punktionsnadel (10) in der Anschlusskanüle (11) durch Klemmen, Schrumpfen und/oder Kleben befestigt ist.

5. Punktionsvorrichtung (1) nach Anspruch 4, **dadurch gekennzeichnet, dass** die Punktionsnadel (10) aus Metall, vorzugsweise aus einer Stahllegierung, und die Anschlusskanüle (11) aus Kunststoff besteht.

6. Punktionsvorrichtung (1) nach Anspruch 2 oder 3, **dadurch gekennzeichnet, dass** die Punktionsnadel (10) und die Anschlusskanüle (11) aus Metall, vorzugsweise aus einer Stahllegierung, bestehen und durch Schweißen, beispielsweise durch Laserschweißen, verbunden sind.

7. Punktionsvorrichtung (1) nach einem der Ansprüche 1 bis 6, **dadurch gekennzeichnet, dass** die Anschlusskanüle (11) im distalen Bereich zwei oder mehrere Übertrittsöffnungen (9, 9') in den Saugkanal (5) aufweist.

8. Punktionsvorrichtung (1) nach Anspruch 7, **dadurch gekennzeichnet, dass** die Übertrittsöffnungen (9, 9') in axialer Richtung hintereinander angeordnet sind.

9. Punktionsvorrichtung (1) nach einem der Ansprüche 1 bis 8, **dadurch gekennzeichnet, dass** die Spülleitung (6) als flexibler Spülschlauch ausgebildet ist, welcher an der Verbindungsstelle (7) zur Punktionskanüle (2) eine Durchmesserverengung, eine Schrumpfschlauchverbindung oder eine Klebeverbindung aufweist.

10. Punktionsvorrichtung (1) nach Anspruch 9, **dadurch gekennzeichnet, dass** die Punktionskanüle (2) ein Griffelement (12) aufweist und in diesem Bereich durch eine Einstichöffnung (13) in das Innere der Spülleitung (6) eintritt und durch den flexiblen Spülschlauch an der Einstichöffnung (13) flüssigkeitsdicht abgedichtet ist.

## Claims

1. A puncturing device (1) for taking organic samples, for example an egg cell for in-vitro fertilization, comprising a puncturing cannula (2) tapering to a needle tip (3), wherein the lumen of the puncturing cannula (2) is configured as a suction channel (5), and comprising a flushing line (6) which serves to supply a flushing agent, wherein a single-lumen puncturing area (A) is formed adjacent to the needle tip (3) of the puncturing cannula (2), in which puncturing area the puncturing cannula has a single lumen, which, starting from a connecting site (7) where the flushing line (6) is attached in a fluid-tight manner to the puncturing cannula (2), is followed by a double-lumen area (B), wherein at the distal end of the flushing line (6) at least one transfer opening (9) is provided in the suction channel (5) of the puncturing cannula (2), **characterised in that** the puncturing cannula (2) has a smaller diameter in the puncturing area (A) than in the proximally adjoining double-lumen area (B), wherein the at least one transfer opening (9) is disposed at the distal end of the double-lumen area (B).

2. The puncturing device (1) according to claim 1, **characterised in that** the puncturing cannula (2) substantially consists of a thin puncturing needle (10) with the needle tip (3) and a proximally adjoining connecting cannula (11), wherein the proximal end of the puncturing needle (10) projects into the connecting cannula (11).

3. The puncturing device (1) according to claim 2, **characterised in that** the outer diameter of the puncturing needle (10) is adapted to the inner diameter of the connecting cannula (11).

4. The puncturing device (1) according to claim 2 or 3, **characterised in that** the proximal end of the puncturing needle (10) is attached in the connecting cannula (11) by clamping, shrinking and/or adhesive bonding.

5. The puncturing device (1) according to claim 4, **characterised in that** the puncturing needle (10) is made of metal, preferably of a steel alloy, and the connecting cannula (11) is made of a plastic material.

6. The puncturing device (1) according to claim 2 or 3, **characterised in that** the puncturing needle (10) and the connecting cannula (11) is made of metal, preferably of a steel alloy, and are joined by welding, for example by laser welding.

7. The puncturing device (1) according to any one of claims 1 to 6, **characterised in that** in the distal area the connecting cannula (11) has two or more transfer openings (9, 9') into the suction channel (5).

8. The puncturing device (1) according to claim 7, **characterised in that** the transfer openings (9, 9') are disposed one behind the other in an axial direction.

9. The puncturing device (1) according to any one of claims 1 to 8, **characterised in that** the flushing line (6) is configured as a flexible flushing tube, which at the connecting site (7) to the puncturing cannula (2) has a reduced diameter, a shrink-on connection or a bonded connection.

10. The puncturing device (1) according to claim 9, **characterised in that** the puncturing cannula (2) has a grip element (12) and enters into the interior of the flushing line (6) in this area via a puncture (13) and is sealed in a fluid-tight manner by the flexible flushing tube at the puncture (13).

## Revendications

1. Dispositif de ponction (1) pour le prélèvement d'échantillons organiques, en particulier d'un ovule pour la fécondation in vitro, comprenant une canule de ponction (2) se terminant par une pointe d'aiguille (3), le lumen de la canule de ponction (2) étant réalisé sous la forme d'un canal d'aspiration (5), ainsi qu'une conduite de lavage (6) servant à l'introduction d'un agent de lavage,
dispositif dans lequel, à la suite de la pointe d'aiguille (3) de la canule de ponction (2) est formée une zone de ponction monolumen (A) dans laquelle la canule de ponction ne comporte qu'un seul lumen, à laquelle se raccorde, à partir d'un emplacement de liaison (7) au niveau duquel la conduite de lavage (6) est fixée de façon étanche aux fluides à la canule de ponction (2), une zone (B) à double lumen, et, à l'extrémité distale de la conduite de lavage (6) est prévue au moins une ouverture de passage (9) dans le canal d'aspiration (5) de la canule de ponction (2), **caractérisé en ce que**
la canule de ponction (2) a, dans la zone de ponction (A), un diamètre inférieur à son diamètre dans la zone (B) à double lumen s'y raccordant côté proximal, l'ouverture de passage (9) étant située à l'extrémité distale de la zone (B) à double lumen.

2. Dispositif de ponction (1) conforme à la revendication 1,
**caractérisé en ce que**
la canule de ponction (2) est essentiellement constituée d'une aiguille de ponction mince (10) équipée la pointe d'aiguille (3) et d'une canule de liaison (11) s'y raccordant côté proximal, l'extrémité proximale de l'aiguille de ponction (10) pénétrant dans la canule de liaison (11).

3. Dispositif de ponction (1) conforme à la revendication 2,
**caractérisé en ce que**
le diamètre extérieur de l'aiguille de ponction (10) est adapté au diamètre intérieur de la canule de liaison (11).

4. Dispositif de ponction (1) conforme à la revendication 2 ou 3,
**caractérisé en ce que**
l'extrémité proximale de l'aiguille de ponction (10) est fixée à la canule de liaison (11) par serrage, rétraction, et/ou collage.

5. Dispositif de ponction (1) conforme à la revendication 4,
**caractérisé en ce que**
l'aiguille de ponction (10) est réalisée en métal, de préférence en un alliage d'acier et la canule de liaison (11) est réalisée en matériau synthétique.

6. Dispositif de ponction (1) conforme à la revendication 2 ou 3,
**caractérisé en ce que**
l'aiguille de ponction (10) et la canule de liaison (11) sont réalisées en métal, de préférence en un alliage d'acier et sont reliées par soudage, par exemple par soudage laser.

7. Dispositif de ponction (1) conforme à l'une des revendications 1 à 6,
**caractérisé en ce que**
la canule de liaison (11) comporte dans sa zone distale, deux ou un plus grand nombre d'ouvertures de passage (9, 9') dans le canal d'aspiration (5).

8. Dispositif de ponction (1) conforme à la revendication 7,
**caractérisé en ce que**
les ouvertures de passage (9, 9') sont situées les unes derrière les autres en direction axiale.

9. Dispositif de ponction (1) conforme à l'une des revendications 1 à 8,
**caractérisé en ce que**
la conduite de lavage (6) est réalisée sous la forme d'un tuyau de lavage flexible qui présente, au niveau de l'emplacement de liaison (7) avec la canule de ponction (2) une diminution de diamètre, une liaison de tuyau par rétraction ou une liaison par collage.

10. Dispositif de ponction (1) conforme à la revendication 9,
**caractérisé en ce que**
la canule de ponction (2) comporte un élément de préhension (12) et pénètre dans cette zone par une ouverture de piqûre (13) à la partie interne de la conduite de lavage (6) et est rendue étanche aux fluides par le tuyau de lavage flexible au niveau de l'ouverture de piqûre (13).
